# EUROPEAN PATENT APPLICATION

(11) **EP 3 178 373 A1**
(43) Date of publication of application: **14.06.2017**
(21) Application number: 16838831.2
(22) Date of filing: 22.03.2016
(51) Int. Cl.: A61B 1/12, G02B 23/24

(54) **ENDOSCOPE REPROCESSOR AND LEAK TEST METHOD FOR ENDOSCOPE REPROCESSOR**

(30) Priority: 26.08.2015 JP 2015167099
(71) Applicant: Olympus Corporation, Tokyo 192-8507 (JP)
(72) Inventor: KAWACHI, Shinichiro, Hachioji-shi Tokyo 192-8507 (JP)
(74) Representative: Schicker, Silvia
(86) International application number: PCT/JP2016/059009
(87) International publication number: WO 2017/033483

(57) **Abstract**

An endoscope reprocessor includes a concave treatment tank 11 in which an endoscope is disposed, a lid 21 that covers the treatment tank 11, an air-open conduit 31 that is connected with the treatment tank 11 and opens the treatment tank 11 to air, a gas filter connection part 35 disposed on the air-open conduit 31, an on-off valve 34 disposed closer to the treatment tank than the gas filter connection part 35 on the air-open conduit 31, a pressure sensor 41 that detects an internal pressure of the treatment tank 11, a pressurizing unit 51 that is disposed closer to the treatment tank than the on-off valve 34 on the air-open conduit 31 or connected with the treatment tank 11 and pressurizes the treatment tank 11 even when the on-off valve 34 is closed, and a control unit 61 that controls the on-off valve 34 and pressurizing unit 51.

## Description

### Technical Field

The present invention relates to an endoscope reprocessor, and a leak test method of an endoscope reprocessor.

### Background Art

As disclosed in, for example, Japanese Patent Application Laid-Open Publication No. 2015-70947, a conventional endoscope cleaning/disinfecting apparatus performs processing such as cleaning/disinfecting on a used and contaminated endoscope. The endoscope cleaning/disinfecting apparatus sets the endoscope in a treatment tank, closes a lid provided to an upper part of the treatment tank, causes the treatment tank to store medicinal solution, and performs cleaning/disinfecting of the endoscope.

In the endoscope cleaning/disinfecting apparatus, a packing is attached to a peripheral part of the lid to prevent leakage of medicinal solution smell or liquid from the treatment tank when the lid is closed.

However, in order to prevent leakage of fluid from the treatment tank, the conventional endoscope cleaning/disinfecting apparatus requires cumbersome work of checking the packing attached to the lid such as check of temporal degradation of the packing or an attachment failure of the packing by a user.

Thus, the present invention is intended to provide an endoscope reprocessor and a leak test method of an endoscope reprocessor, which are capable of detecting fluid leakage from the treatment tank.

### Disclosure of Invention

### Means for Solving the Problem

An endoscope reprocessor according to an aspect of the present invention includes a concave treatment tank in which an endoscope is disposed, a lid that covers the treatment tank, an air-open conduit that is connected with the treatment tank and opens the treatment tank to air, a gas filter connection part disposed on the air-open conduit, an on-off valve disposed closer to the treatment tank than the gas filter connection part on the air-open conduit, a pressure sensor that detects an internal pressure of the treatment tank, a pressurizing unit that is disposed closer to the treatment tank than the on-off valve on the air-open conduit or connected with the treatment tank and pressurizes the treatment tank even when the on-off valve is closed, and a control unit that controls the on-off valve and the pressurizing unit.

A leak test method of an endoscope reprocessor according to an aspect of the present invention includes a first step of closing the on-off valve, a second step of pressurizing the treatment tank by driving the pressurizing unit after the first step, a third step of detecting an internal pressure of the treatment tank by the pressure sensor after the second step, a fourth step of opening the on-off valve after the third step, a fifth step of detecting an internal pressure of the treatment tank by the pressure sensor after the fourth step, a sixth step of determining sealing of the treatment tank by comparing an internal pressure measured at the third step to a first reference pressure, and a seventh step of determining sealing between the gas filter connection part and a gas filter by comparing an internal pressure measured at the sixth step to a second reference pressure.

### Brief Description of the Drawings

Fig. 1 is a diagram illustrating the configuration of a main part of an endoscope reprocessor according to an embodiment of the present invention;
Fig. 2 is a flowchart illustrating the process of leak test processing of the endoscope reprocessor according to the embodiment of the present invention;
Fig. 3 is a flowchart illustrating the process of the leak test processing of the endoscope reprocessor according to the embodiment of the present invention; and
Fig. 4 is a graph illustrating a relation between an elapsed time and an internal pressure of the endoscope reprocessor according to the embodiment of the present invention.

### Best Mode for Carrying Out the Invention

An embodiment of the present invention will be described below with reference to the accompanying drawings.

### (Configuration)

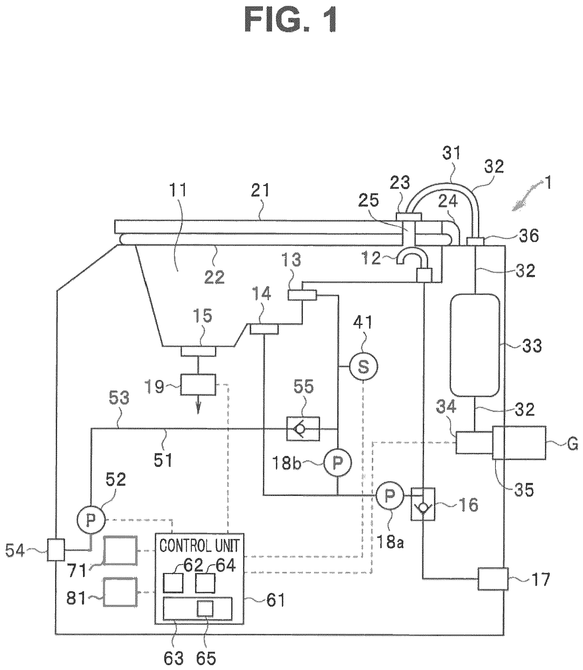

Fig. 1 is a diagram illustrating the configuration of a main part of an endoscope reprocessor 1 according to the embodiment of the present invention. In Fig. 1, a solid line connecting respective components represents a fluid conduit, and a dashed line connecting respective components represents an electric signal line.

The endoscope reprocessor 1 is configured to include a treatment tank 11, a lid 21, an air-open conduit 31, a pressure sensor 41, a pressurizing unit 51, and a control unit 61.

The treatment tank 11 is formed in a concave shape, and an endoscope not illustrated can be disposed therein. The treatment tank 11 can store therein liquid such as water, cleaning liquid, alcohol, or disinfecting liquid. The treatment tank 11 is configured to include a nozzle 12, a connector 13, a circulation port 14, and a drain port 15.

The nozzle 12 is connected with an external hydrant (not illustrated) through a check valve 16 and a hydrant connection port 17.

The check valve 16 is configured to flow fluid from the hydrant connection port 17 to the treatment tank 11, and is configured not to flow fluid from the treatment tank 11 to the hydrant connection port 17.

The nozzle 12 can supply, to the treatment tank 11, water taken in through the hydrant connection port 17. The nozzle 12 is connected with a liquid pump 18a and the circulation port 14. Liquid in the treatment tank 11, which is taken in through the circulation port 14 is pressurized by the liquid pump 18a, and can be supplied to the treatment tank 11 through the nozzle 12 again. The nozzle 12 can supply, to the treatment tank 11, liquid such as cleaning liquid supplied from an internal tank (not illustrated).

The connector 13 is connected with a pipe sleeve of the endoscope (not illustrated) through a connection tube (not illustrated). The connector 13 is connected with a liquid pump 18b. During processing such as cleaning/disinfecting of the endoscope, the endoscope reprocessor 1 causes the liquid pump 18b to drive to feed, to an endoscope conduit through the connector 13, liquid in the treatment tank 11, which is taken in through the circulation port 14.

When the processing such as cleaning/disinfecting is performed on the endoscope, the circulation port 14 can take in the liquid stored in the treatment tank 11.

The nozzle 12, the connector 13, and the circulation port 14 are connected with an air feeding conduit 53 connected with a gas pump 52 to be described later.

The drain port 15 is connected with a drain valve 19, and can eject the liquid stored in the treatment tank 11. When the drain valve 19 is opened, the liquid stored in the treatment tank 11 is externally ejected through the drain port 15. When the drain valve 19 is closed, the treatment tank 11 is externally disconnected so that the liquid in the treatment tank 11 is not externally ejected and gas in the treatment tank 11 pressurized by the gas pump 52 is not externally ejected.

The lid 21 is configured to include a packing 22 and a treatment tank connection port 23. The lid 21 is formed to cover the treatment tank 11. The lid 21 is provided to be freely opened and closed to the treatment tank 11 through a hinge 24. The endoscope reprocessor 1 allows the endoscope to be disposed in the treatment tank 11 when the lid 21 is opened.

The packing 22 is configured to be made of material such as rubber or synthetic resin. The packing 22 is provided around a periphery of the lid 21. When the lid 21 is closed, the packing 22 is pressed in contact with a periphery of an upper part of the treatment tank 11, thereby preventing leaking of the liquid or the gas in the treatment tank 11. When used and depleted, the packing 22 can be removed and replaced.

The treatment tank connection port 23 is connected with the air-open conduit 31. The treatment tank connection port 23 is configured to include a hole 25 penetrating through the lid 21, and causes the air-open conduit 31 to communicate with inside of the treatment tank 11.

A gas filter G is detachably attached to a gas filter connection part 35. The air-open conduit 31 is connected with the treatment tank 11 through the lid 21, and the treatment tank 11 is open to air by opening an exhaust valve 34.

An exhaust tube 32 that is part of the air-open conduit 31 has one of end parts connected with the treatment tank connection port 23 provided in an upper part of the lid 21, a body part placed above the lid 21, and the other end part connected with a body connection port 36. The exhaust tube 32 is configured in a shape such as an accordion shape, and is configured to be freely expanded and contracted, and freely bent in accordance with an opening and closing operation on the lid 21.

A chamber 33 may be provided at a middle position of the air-open conduit 31. The chamber 33 is configured in a shape of a hollow box. The chamber 33 is disposed, on the air-open conduit 31, closer to the treatment tank 11 than the exhaust valve 34 that is an on-off valve. The chamber 33 is connected with the exhaust valve 34. When the inside of the treatment tank 11 is pressurized by the pressurizing unit 51, the chamber 33 causes air in the chamber 33 to buffer an abrupt increase of pressure to reduce a load provided to the treatment tank 11 or the air feeding conduit 53.

The exhaust valve 34 is provided on the air-open conduit 31. The exhaust valve 34 that is an on-off valve may be configured to be an electromagnetic valve that can be freely opened and closed. The exhaust valve 34 that is an on-off valve is disposed, on the air-open conduit 31, closer to the treatment tank 11 than the gas filter connection part 35. The exhaust valve 34 is connected with the gas filter connection part 35. The exhaust valve 34 is electrically connected with the control unit 61 and operable to be opened and closed in accordance with a control signal received from the control unit 61.

The gas filter connection part 35 is disposed on the air-open conduit 31 and can be connected with the gas filter G.

The gas filter G can filter gas having medicinal solution smell inside the treatment tank 11 to remove the medicinal solution smell, and can externally eject the gas. The gas filter G is configured to have a predetermined ventilation resistance.

The pressure sensor 41 can detect the internal pressure of the treatment tank 11. The pressure sensor 41 is connected with the treatment tank 11 through the connector 13. The pressure sensor 41 is electrically connected with the control unit 61. The pressure sensor 41 can detect the internal pressure of the treatment tank 11, and can transmit, to the control unit 61, information on the internal pressure of the treatment tank 11 as a result of the detection.

Note that the pressure sensor 41 is also used to detect the pressure of liquid being fed to the endoscope conduit through the connector 13 when the processing such as cleaning/disinfecting is performed on the endoscope.

The pressurizing unit 51 is configured to be able to pressurize the treatment tank 11. The pressurizing unit 51 is configured to include the gas pump 52 that is an air feeding unit and the air feeding conduit 53. The pressurizing unit 51 is disposed to be connected with the treatment tank 11. Note that the pressurizing unit 51 may be configured to be disposed closer to the treatment tank 11 than the exhaust valve 34 on the air-open conduit 31.

The gas pump 52 that is an air feeding unit can pressurize gas. The gas pump 52 is connected with an air-open port 54. The gas pump 52 is electrically connected with the control unit 61, and is driven in accordance with a control signal received from the control unit 61.

The air feeding conduit 53 is a conduit connecting the gas pump 52 that is an air feeding unit and the treatment tank 11. More specifically, the air feeding conduit 53 connects the gas pump 52 and a check valve 55. Then, the air feeding conduit 53 connects the nozzle 12, the connector 13, and the circulation port 14, which are provided in the treatment tank 11, and the check valve 55.

The check valve 55 is configured to flow fluid from the gas pump 52 to the treatment tank 11 but not to flow fluid from the treatment tank 11 to the gas pump 52.

When the processing such as cleaning/disinfecting is performed on the endoscope, the exhaust valve 34 is opened, and the gas pump 52 feeds air to the endoscope conduit through the connector 13 and is used to remove water from the endoscope conduit. When a leak test of the treatment tank 11 is performed, in the present embodiment, the exhaust valve 34 is closed, and the gas pump 52 takes in air through the air-open port 54, pressurizes the air, feeds the air to the treatment tank 11 through the air feeding conduit 53, and pressurizes the treatment tank 11. That is, the gas pump 52 that is a pressurizing unit can pressurize the treatment tank 11 even when the exhaust valve 34 is closed.

The control unit 61 is configured to include a central processing unit (hereinafter referred to as a "CPU") 62, a ROM 63, and a RAM 64. The CPU 62 can execute various kinds of computer programs stored in the ROM 63. A function of the CPU 62 is achieved by executing the various kinds of programs. The ROM 63 stores therein a program for the processing such as cleaning/disinfecting on the endoscope, and a program related to the leak test.

The control unit 61 is connected with the drain valve 19, the exhaust valve 34, the pressure sensor 41, and the gas pump 52, controls, based on processing of programs, the drain valve 19, the exhaust valve 34, and the gas pump 52, so as to acquire information on the internal pressure of the treatment tank 11 from the pressure sensor 41.

The control unit 61 is configured to include a determination unit 65.

The determination unit 65 is configured by a program stored in the ROM 63. The determination unit 65 is a processing unit that determines fluid leak by comparing a change in the internal pressure detected by the pressure sensor 41 to a reference pressure.

A display unit 71 is electrically connected with the control unit 61. When, by the leak test processing, air leakage through the packing 22 is detected or an attachment failure of the gas filter G is detected, the display unit 71 can display a result of the detection in accordance with a control signal from the control unit 61 to give notification to a user.

A sound generating unit 81 is electrically connected with the control unit 61. When, by the leak test processing, air leakage through the packing 22 is detected or an attachment failure of the gas filter G is detected, the display unit 71 can output warning sound in accordance with a control signal from the control unit 61 to give notification to the user.

### (Effect)

The following describes leak test processing of the endoscope reprocessor 1.

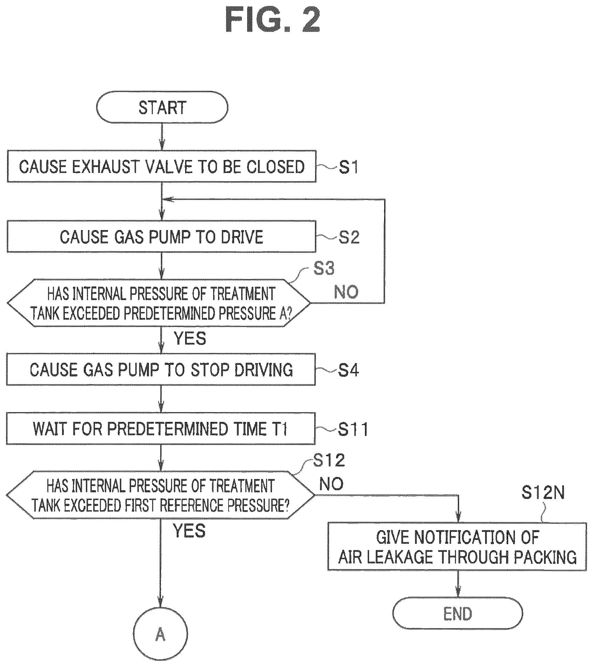

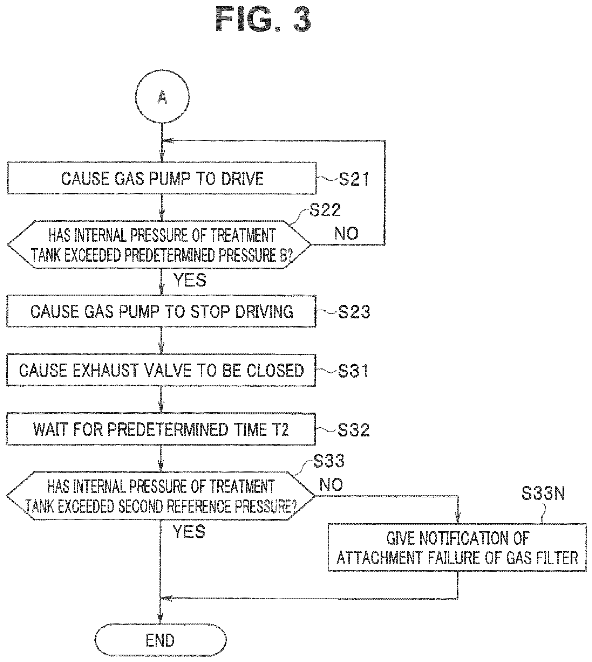

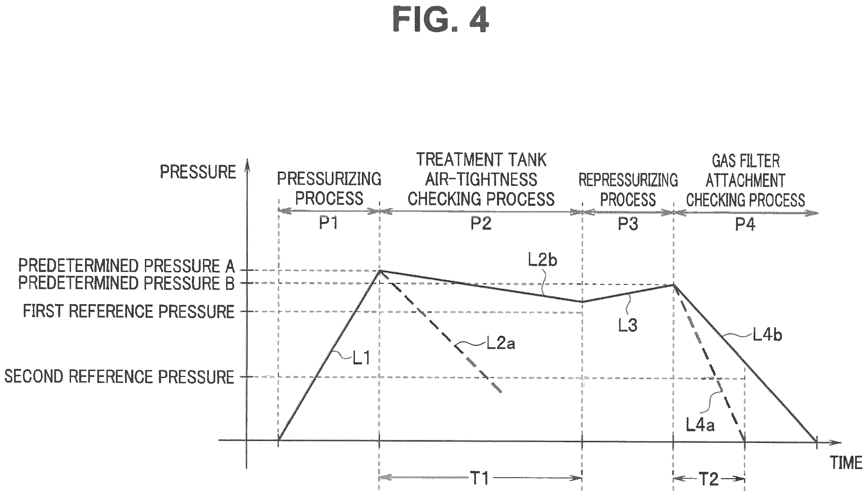

Figs. 2 and 3 are flowcharts illustrating the flow of the leak test processing of the endoscope reprocessor 1 according to the embodiment of the present invention. Fig. 4 is a graph illustrating a relation between an elapsed time and the internal pressure of the endoscope reprocessor 1 according to the embodiment of the present invention.

When the user closes the lid 21 of the endoscope reprocessor 1 and provides an instruction to start the leak test through an operation portion (not illustrated), the CPU 62 reads in a program for the leak test from the ROM 63, loads the program onto the RAM 64, and starts processing of the program.

The control unit 61 causes the exhaust valve 34 to be closed (S1). The control unit 61 transmits, to the exhaust valve 34, a control signal for causing the exhaust valve 34 to be closed. The exhaust valve 34 receives the control signal from the control unit 61 and closes the exhaust valve 34. When the drain valve 19 is open, the control unit 61 transmits, to the drain valve 19, a control signal for causing the drain valve 19 to be closed, thereby causing the drain valve 19 to be closed. Accordingly, the treatment tank 11 is cut off from air to be a closed state.

The control unit 61 causes the gas pump 52 to drive (S2). The control unit 61 transmits, to the gas pump 52, a control signal for causing the gas pump 52 to drive. The gas pump 52 receives the control signal from the control unit 61, takes in gas from air, feeds the gas into the treatment tank 11 through the air feeding conduit 53, and pressurizes the inside of the treatment tank 11.

The control unit 61 determines whether the internal pressure of the treatment tank 11 has exceeded a predetermined pressure A (S3). At S3, the control unit 61 acquires information on the internal pressure of the treatment tank 11 from the pressure sensor 41. The control unit 61 determines whether the internal pressure of the treatment tank 11 has exceeded the predetermined pressure A. If it is determined that the internal pressure of the treatment tank 11 is not higher than the predetermined pressure A (NO at S3), the process returns to S2. If it is determined that the internal pressure of the treatment tank 11 has exceeded the predetermined pressure A (YES at S3), the process proceeds to S4. Through S2 and S3, the inside of the treatment tank 11 is pressurized to the predetermined pressure A (L1 illustrated in Fig. 4).

The predetermined pressure A is set in advance, as a pressure that allows detection of a pressure decrease when air leakage through the packing 22 is present, within the range of durability of the endoscope reprocessor 1.

The control unit 61 causes the gas pump 52 to stop driving (S4).

The processing at S1 to S4 is processing of a pressurizing process P1.

The control unit 61 waits for a predetermined time T1 (S11). Since the treatment tank 11 is in a closed state being cut off from air, a reduced amount of the internal pressure of the treatment tank 11 during the predetermined time T1 is zero or a little amount with no air leakage through the packing 22.

The predetermined time T1 is set in advance as a time that allows detection of a pressure decrease due to air leakage through the packing 22, and is, for example, any time between 20 seconds to 40 seconds.

After the predetermined time T1 has elapsed, the control unit 61 determines whether the internal pressure of the treatment tank 11 has exceeded a first reference pressure (S12). At S12, the control unit 61 acquires information on the internal pressure of the treatment tank 11 from the pressure sensor 41. The control unit 61 determines whether the internal pressure of the treatment tank 11 has exceeded the first reference pressure. If it is determined that the internal pressure of the treatment tank 11 is not higher than the first reference pressure (NO at S12; L2a illustrated in Fig. 4), air leakage through the packing 22 is detected, and the process proceeds to S12N. If it is determined that the internal pressure of the treatment tank 11 has exceeded the first reference pressure (YES at S12; L2b illustrated in Fig. 4), no air leakage through the packing 22 is detected, and the process proceeds to S21.

The first reference pressure is set in advance to be a pressure lower than a pressure when no air leakage through the packing 22 is present and higher than a pressure when air leakage through the packing 22 is present after the predetermined time T1 has elapsed since the internal pressure of the treatment tank 11 was increased to the predetermined pressure A.

If air leakage through the packing 22 is detected (NO at S12), the control unit 61 gives notification of air leakage through the packing 22 (S12N). At S12N, the control unit 61 causes the display unit 71 to display the air leakage through the packing 22 or causes the sound generating unit 81 to generate the warning sound, thereby notifying the user of the air leakage through the packing 22. After the notification of the air leakage through the packing 22, the control unit 61 ends the leak test processing.

The processing at S11 to S12N is processing of a treatment tank air-tightness checking process P2.

The pressurizing process P1 and the treatment tank air-tightness checking process P2 described above allow detection of whether a leak of gas from the packing 22 is present.

If no air leakage through the packing 22 is detected (YES at S12), the control unit 61 causes the gas pump 52 to drive (S21 illustrated in Fig. 3). When no air leakage through the packing 22 is detected, the internal pressure of the treatment tank 11 still potentially decreases due to a small amount of air leakage until the predetermined time T1 has elapsed. At S21, the control unit 61 transmits, to the gas pump 52, a control signal for causing the gas pump 52 to drive, thereby causing the gas pump 52 to drive to pressurize the treatment tank 11 again.

The control unit 61 determines whether the internal pressure of the treatment tank 11 has exceeded a predetermined pressure B (S22). At S22, the control unit 61 acquires information on the internal pressure of the treatment tank 11 from the pressure sensor 41. The control unit 61 determines whether the internal pressure of the treatment tank 11 has exceeded the predetermined pressure B. If it is determined that the internal pressure of the treatment tank 11 is not higher than the predetermined pressure B (NO at S22), the process returns to S21. If it is determined that the internal pressure of the treatment tank 11 has exceeded the predetermined pressure B (YES at S22), the process proceeds to S23. Through S21 and S22, the treatment tank 11 is pressurized to the predetermined pressure B (L3 illustrated in Fig. 4).

The predetermined pressure B is set in advance, as a pressure that allows detection of a pressure decrease when the gas filter G is not attached, within the range of durability of the endoscope reprocessor 1. Note that the predetermined pressure B may be set to a value identical to the value of the predetermined pressure A.

The control unit 61 causes the gas pump 52 to stop driving (S23).

The processing at S21 to S23 is processing of repressurizing process P3.

The control unit 61 causes the exhaust valve 34 to open (S31). The control unit 61 transmits, to the exhaust valve 34, a control signal for causing the exhaust valve 34 to open. The exhaust valve 34 receives the control signal from the control unit 61 and opens the exhaust valve 34.

The control unit 61 waits for a predetermined time T2 (S32). Since the gas filter G has a ventilation resistance, the internal pressure of the treatment tank 11 decreases slower when the gas filter G is attached than when the gas filter G is removed.

The predetermined time T2 is set in advance to allow detection of an attachment failure of the gas filter G through decrease of the internal pressure of the treatment tank 11, and is, for example, any time between one second to three seconds.

The control unit 61 determines whether the internal pressure of the treatment tank 11 has exceeded a second reference pressure (S33). At S33, the control unit 61 acquires information on the internal pressure of the treatment tank 11 from the pressure sensor 41. The control unit 61 determines whether the internal pressure of the treatment tank 11 has exceeded the second reference pressure. If it is determined that the internal pressure of the treatment tank 11 has not exceeded the second reference pressure (NO at S33; L4a illustrated in Fig. 4), it is detected that the gas filter G is not attached, and the process proceeds to S33N. If it is determined that the internal pressure of the treatment tank 11 has exceeded the second reference pressure (YES at S33; L4b illustrated in Fig. 4), it is detected that the gas filter G is attached, and the process ends.

The second reference pressure varies depending on the capacity of a space including the treatment tank 11 and the ventilation resistance of the gas filter G, and is set in advance to be a pressure lower than a pressure when the gas filter G is attached and higher than a pressure when the gas filter G is not attached after the predetermined time T2 has elapsed since the internal pressure of the treatment tank 11 was increased to the predetermined pressure B.

If it is detected that the gas filter G is not attached (NO at S33), the control unit 61 gives notification of an attachment failure of the gas filter G (S33N). At S33N, the control unit 61 causes the display unit 71 to display the attachment failure of the gas filter G or causes the sound generating unit 81 to generate the warning sound, thereby notifying the user of the attachment failure of the gas filter G. After the notification of the attachment failure of the gas filter G, the control unit 61 causes the leak test processing to end.

The processing at S31 to S33 is processing of a gas filter attachment checking process P4.

The repressurizing process P3 and gas filter attachment checking process P4 described above allows detection of an attachment failure of the gas filter G.

The above-described embodiment can provide the endoscope reprocessor 1 and a leak test method of the endoscope reprocessor 1, which are capable of detecting whether gas leakage from the packing 22 is present, detecting an attachment failure of the gas filter G, and thus detecting fluid leakage from the treatment tank 11.

Note that the function of the determination unit 65 is achieved by executing a program recorded in the ROM 63 in the embodiment, but may be achieved by providing a circuit.

Note that the steps in each procedure in the embodiment may be executed in a different execution order, may be simultaneously executed, or may be executed in a different order at each execution, without losing the characteristics of the procedure. In addition, all or part of the steps of each procedure in the embodiment may be achieved by hardware.

Note that the treatment tank connection port 23 connected with the exhaust tube 32 is provided to the lid 21 in the embodiment, but may be provided to a sidewall of the treatment tank 11. In this case, the treatment tank connection port 23 may be provided to, for example, an upper part of the sidewall of the treatment tank 11, thereby preventing the liquid inside of the treatment tank 11 from flowing into the air-open conduit 31. With this configuration, the exhaust tube 32 is provided in the endoscope reprocessor 1, thereby achieving a simple appearance of an upper part of the lid 21.

Note that air is taken in by the gas pump 52 so that the treatment tank 11 is pressurized by gas in the embodiment, but, a liquid pump may be included to take in water through an external hydrant so that the treatment tank 11 is pressurized by liquid.

Note that the endoscope reprocessor 1 is a device that performs reprocessing on contaminated endoscope or endoscope accessory. The reprocessing is not particularly limited, but may be any of rinsing with water, cleaning of contamination such as organic substance, disinfecting of predetermined microorganisms to be ineffective, sterilizing to remove or kill all microorganisms, or a combination of these processing.

The present invention is not limited to the above-described embodiment, but, for example, various kinds of changes and modifications are applicable without departing from the scope of the present invention.

The present invention can provide an endoscope reprocessor capable of detecting fluid leakage from a treatment tank, and a leak test method of the endoscope reprocessor.

The present application is filed, claiming priority based on Japanese Patent Application No. 2015-167099 filed to Japan on August 26, 2015, the content of which is incorporated in the specification and claims of this application by reference thereto.

## Claims

1. An endoscope reprocessor comprising:
a concave treatment tank in which an endoscope is disposed;
a lid that covers the treatment tank;
an air-open conduit that is connected with the treatment tank and opens the treatment tank to air;
a gas filter connection part disposed on the air-open conduit;
an on-off valve disposed, on the air-open conduit, closer to the treatment tank than the gas filter connection part;
a pressure sensor that detects an internal pressure of the treatment tank;
a pressurizing unit that is disposed closer to the treatment tank than the on-off valve on the air-open conduit or connected with the treatment tank, and pressurizes the treatment tank even when the on-off valve is closed; and
a control unit that controls the on-off valve and the pressurizing unit.

2. The endoscope reprocessor according to claim 1, comprising a determination unit that determines leak by comparing the internal pressure detected by the pressure sensor to a reference pressure.

3. The endoscope reprocessor according to claim 1, wherein the pressurizing unit includes:
an air feeding unit that feeds gas; and
an air feeding conduit that connects the air feeding unit and the treatment tank.

4. The endoscope reprocessor according to claim 1, wherein the air-open conduit includes a chamber disposed closer to the treatment tank than the on-off valve.

5. The endoscope reprocessor according to claim 1, wherein the air-open conduit includes an exhaust tube connected with the treatment tank through the lid.

6. A leak test method of an endoscope reprocessor using the endoscope reprocessor according to claim 1, the method comprising:
closing the on-off valve;
pressurizing, after the closing, the treatment tank by driving the pressurizing unit;
detecting, after the pressurizing, a first internal pressure of the treatment tank by the pressure sensor;
opening the on-off valve after the detecting;
detecting, after the opening, a second internal pressure of the treatment tank by the pressure sensor;
determining sealing of the treatment tank by comparing an internal pressure measured by the detection of the first internal pressure to a first reference pressure; and
determining sealing between the gas filter connection part and a gas filter by comparing an internal pressure measured by the detection of the second internal pressure to a second reference pressure.

7. The leak test method of an endoscope reprocessor according to claim 6, comprising, before the opening after the detection of the first internal pressure, pressurizing the treatment tank and the air-open conduit by driving the pressurizing unit.
